# EUROPEAN PATENT APPLICATION

(11) **EP 1 598 068 A1**
(43) Date of publication of application: **23.11.2005**
(21) Application number: 05002518.8
(22) Date of filing: 12.12.2000
(51) Int. Cl.: A61K 31/428, C07D 417/14, A61P 25/18, A61P 25/24

(54) **Antipsychotic heterocycle compounds**

(30) Priority: 17.12.1999 US 172200 P
(62) Divisional of application: 00992008.3
(71) Applicant: Bristol-Myers Squibb Company, Princeton, NJ 08543 (US)
(72) Inventor: Mattson, Ronald J., Meriden Connecticut 06451 (US); Yevich, Joseph P., Southington Connecticut 06489 (US); Yuan, Jun, Guilford Connecticut 06437 (US); Eison, Arlene S., Killingworth Connecticut 06419 (US); Denhart, Derek, Durham, CT 06422 (US)
(74) Representative: Vossius & Partner

(57) **Abstract**

Compounds of Formula (I) are useful antipsychotic and antidepressant agents demonstrating potent inhibition of 5-HT reuptake and dopamine D2 receptor antogonism. 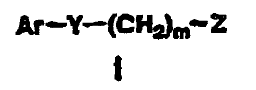 wherein:
Ar is 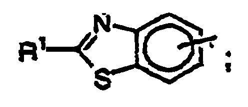
Y is selected from oxygen and sulfur;
Z is II:

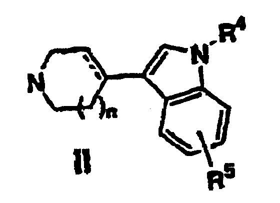

## Description

### CROSS REFERENCE TO RELATED APPLICATIONS

This non-provisional application claims priority from provisional application USSN 60/172,200 filed December 17, 1999.

### BACKGROUND OF THE INVENTION

This invention pertains to cyclic amino derivatives having psychotropic and bio-affecting properties and to their preparation and use in treating patients suffering from or susceptible to psychosis, acute mania, mild anxiety states, or depression in combination with psychotic episodes by the administration of these cyclic amino derivatives. More specifically, the invention is concerned with the medicinal use of compounds having benzene or benzothiazole rings linked by sidechains to the nitrogen atom of 4-substituted-1,2,5,6-tetrahydropyridine and -piperidine moieties. These compounds possess unique dopaminergic and serotonergic profiles that make them useful in the treatment of psychosis and other mental illnesses caused by disorders of the dopaminergic and serotonergic systems.

The combination of a serotonin reuptake inhibitor, such as fluoxetine, with a dopaminergic antipsychotic agent, such as olanzapine, has been described as an improved treatment for psychosis, (European Patent Application 830864, published Sept 22, 1997).

The preparation and use of tetrahydropyridinyl- and piperidinylindoles, 1, and related compounds as serotonin 5-HT_{1F} agonists for the treatment of migraine, allergic rhinitis, and associated diseases has been described. Cf: US Patent 5,521,197 (May 28, 1996), WO Patent Publication No. 9811895 (March 26, 1998) and WO 9806402 (February 19, 1998), and European Patent Application 714894 (June 5, 1996).

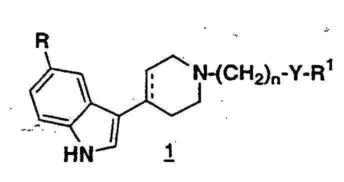

In 1, Y = O, S, or a bond, and R¹ is naphthyl, phenyl, substituted phenyl, halo, alkyl, alkylthio, alkoxy, benzyloxy, OH, CONH₂.

A series of aryloxy propanolamines, including compound 2, was disclosed by Obase, et al., in Chem. Pharm. Bull., 30(2), 474-83, 1982, as antihypertensive agents.

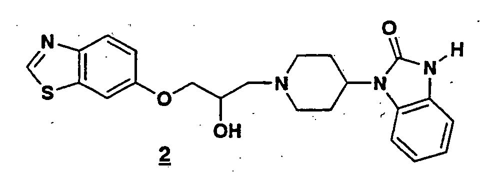

The synthesis and adrenergic β-blocking activity of some non-cyclic amine derivatives; e.g., 3, has been described by Obase, et al., Chem. Pharm. Bull., 26(5), 1443-52, 1978, and by Franke, et al., Arzneim.-Forsch., 30(11), 1831-8, 1980.

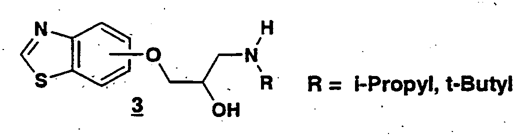

Some derivatives of 4-(1 H-indol-3-yl)-1 piperidine-ethanol derivatives, 4, having antiarrhythmic activity were described by Clemence, et al., in US Patent 4,530,932 (July 23, 1985). 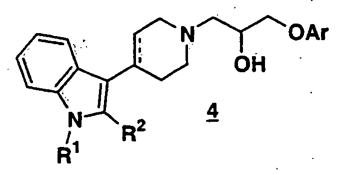 In 4, Ar is aryl or a selected group of heteroaryl moieties not including benzothiazole.

The preparation and use of indane and dihydroindole derivatives of indolylpiperidine compounds, 5, as dopamine D₄ receptor, 5-HT receptor, and 5-HT transporter ligands was described in WO Patent Publication No. 9828293 (July 2, 1998).

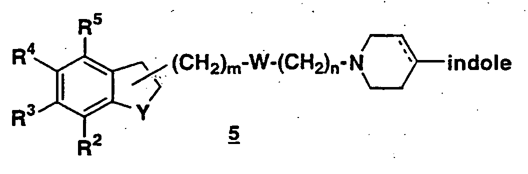

In sum, none of these references suggest the novel compounds of the present invention.

A series of nitrogen-containing heterocycles linked by oxygen to alkanamines comprising, inter alia, compounds of formula 6, were disclosed and claimed for the treatment of conditions related to the reuptake of serotonin and by the 5-HT₁ₐ receptor (US Patent 5,741,789, April 21, 1998, and US Patent 5,627,196, May 6, 1997). In formula 6, D represents a nitrogen-containing residue that completes 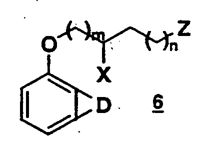 fused pyrrolo, imidazolo, pyrido, pyrazino, pyridazino, or pyrimido moieties.

Z can be 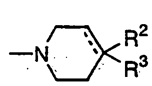 with R² being absent when a double bond is intended or being hydrogen or a substituent, including a benzyl group. R³ is a non-hydrogen substituent that can be indole.

### SUMMARY OF THE INVENTION

In its broadest aspect, the invention is concerned with the use of certain benzene or benzothiazole compounds linked by sidechains to indolyl-1,2,5,6-tetrahydropyridines and -piperidines or substituted 4-benzylpiperidines. These compounds possess a unique dopaminergic and serotonergic profile useful for treating CNS disorders such as psychosis and depression and they conform to Formula I: 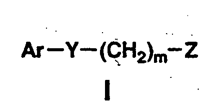 In Formula I:
Ar is selected from 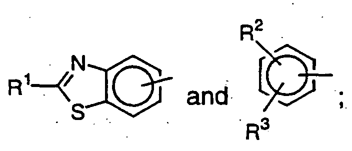
Z is II or III; 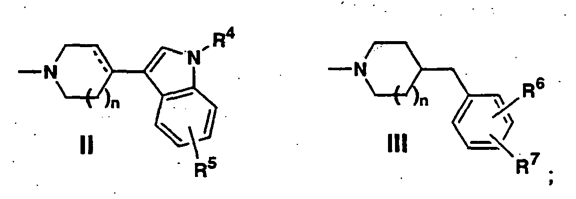
Y is sulfur or oxygen;
R¹ and R⁴ are independently selected from H and lower alkyl;
R², R³, R⁶ and R⁷ are independently selected from H, halogen, and lower alkoxy;
R⁵ is selected from H, halogen, lower alkoxy and cyano;
m is an integer from 2-6;
n is zero or the integer 1 or 2; and
a dotted line represents an optional double bond.

"Halo" or "halogen" refers to fluoride, chloride, bromide or iodide substituents with fluoride, chloride and bromide preferred.

"Lower" refers to an alkyl or alkoxy group having from one to four carbon atoms.

Additionally, compounds of Formula I also encompass all pharmaceutically acceptable acid addition safts and/or solvates thereof. The present invention is also considered to include stereoisomers including geometric as well as optical isomers, e.g. mixtures of enantiomers as well as individual enantiomers and diasteromers, which arise as a consequence or structural asymmetry in certain compounds of the instant series. Separation of the individual isomers is accomplished by application of various methods which are well known to practitioners in the art.

Several classes of Formula I compounds are intended and result from selection among benzene, benzothiazole, and II and III structural moieties. The benzothiazole classes of compounds are novel.

Preferred compounds are those wherein m is 3, n is 1 and Y is oxygen.

Preferred compound examples where Z is Formula II are shown below.

### A. Benzothiazole Derivatives

5-{3-[4-(5-fiuoroindol-3-yl)-1,2,5,6-tetrahydropyrid-1-yl]propoxy}-2-methylbenzothiazole;
5-{3-[4-(5-cyanoindol-3-yl)-1,2,5,6-tetrahydropyrid-1-yl]propoxy}-2-methylbenzothiazole;
5-{3-[4-(5-fluoroindol-3-yl)piperidinyl]propoxy}-2-methylbenzothiazole;
5-{3-[4-(5-cyanoindol-3-yl)piperidinyl]propoxy}-2-methylbenzothiazole;
5-{4-[4-(5-cyanoindol-3-yl)-1,2,5,6-tetrahydropyrid-1-yl]butoxy}-2-methylbenzothiazole;
5-{4-[4-(5-cyanoindol-3-yl)piperidinyl]butoxy}-2-methylbenzothiazole;
5-{3-[4-(5-bromoindol-3-yl)-1,2,5,6-tetrahydropyrid-1-yl]propoxy}-2-methylbenzothiazole;
5-{3-[4-(4-fluoroindol-3-yl)-1,2,5,6-tetrahydropyrid-1-yl]propoxy}-2-methylbenzothiazole;
5-{4-[4-(4-fluoroindol-3-yl)1,2,5,6-tetrahydropyrid-1-yl]butoxy}-2-methylbenzothiazole;
5-{5-[4-(4-fluoroindol-3-yl)-1,2,5,6-tetrahydropyrid-1-yl]pentoxy}-2-methylbenzothiazole;
5-{4-[4-(5-fluoroindol-3-yl)1,2,5,6-tetrahydropyrid-1-yl]butoxy}-2-methylbenzothiazole;
5-{5-[4-(5-fluoroindol-3-yl)-1,2,5,6-tetrahydropyrid-1-yl]pentoxy}-2-methylbenzothiazole;
5-{3-[4-(7-fluoroindol-3-yl)-1,2,5,6-tetrahydropyrid-1-yl]propoxy}-2-methylbenzothiazole;
5-{4-[4-(7-fluoroindol-3-yl)1,2,5,6-tetrahydropyrid-1-yl]butoxy}-2-methylbenzothiazole;
5-{5-[4-(7-fluoroindol-3-yl)-1,2,5,6-tetrahydropyrid-1-yl]pentoxy}-2-methylbenzothiazole;
5-{4-[4-(5-chloroindol-3-yl)1,2,5,6-tetrahydropyrid-1-yl]butoxy}-2-methylbenzothiazole;
5-{5-[4-(5-chloroindol-3-yl)-1,2,5,6-tetrahydropyrid-1-yl]pentoxy}-2-methylbenzothiazole;
5-{3-[4-(6-chloroindol-3-yl)-1,2,5,6-tetrahydropyrid-1-yl]propoxy}-2-methylbenzothiazole;
5-{4-[4-(6-chloroindol-3-yl)1,2,5,6-tetrahydropyrid-1-yl]butoxy}-2-methylbenzothiazole;
5-{5-[4-(6-chloroindol-3-yl)-1,2,5,6-tetrahydropyrid-1-yl]pentoxy}-2-methylbenzothiazole;
5-{3-[4-(6-bromoindol-3-yl)-1,2,5,6-tetrahydropyrid-1-yl]propoxy}-2-methylbenzothiazole;
5-{4-[4-(6-bromoindol-3-yl)1,2,5,6-tetrahydropyrid-1-yl]butoxy}-2-methylbenzothiazole;
5-{5-[4-(6-brompindol-3-yl)-1,2,5,6-tetrahydropyrid-1-yl]pentoxy}-2-methylbenzothiazole;
5-{3-[4-(7-bromoindol-3-yl)-1,2,5,6-tetrahydropyrid-1-yl]propoxy}-2-methylbenzothiazole;
5-{4-[4-(7-bromoindol-3-yl)1,2,5,6-tetrahydropyrid-1-yl]butoxy}-2-methylbenzothiazole;
5-{5-[4-(7-bromoindol-3-yl)-1,2,5,6-tetrahydropyrid-1-yl]pentoxy}-2-methylbenzothiazole;
5-{3-[4-(5-methoxyindol-3-yl)-1,2,5,6-tetrahydropyrid-1-yl]propoxy}-2-methylbenzothiazole;
5-{4-[4-(5-methoxyindol-3-yl)1,2,5,6-tetrahydropyrid-1-yl]butoxy}-2-methylbenzothiazole; and
5-{5-[4-(5-methoxyindol-3-yl)-1,2,5,6-tetrahydropyrid-1-yl]pentoxy}-2-methylbenzothiazole.

### B. Benzene Derivatives

3-[1-(3-phenoxypropyl)-4-piperidinyl]-5-cyanoindole;
3-{1-[3-(2-fluorophenoxy)propyl]-4-piperidinyl}-5-cyanoindole;
3-{1-[3-(4-fluorophenoxy)propyl]-4-piperidinyl}-5-cyanoindole;
3-{1-[3-(2-methoxyphenoxy)propyl]-4-piperidinyl}-5-cyanoindole;
3-{1-[3-(3-methoxyphenoxy)propyl]-4-piperidinyl}-5-cyanoindole;
3-{1-[3-(4-methoxyphenoxy)propyl]-4-piperidinyl}-5-cyanoindole; and
3-{1-[3-(3,4-dimethoxyphenoxy)propyl]-4-piperidinyl}-5-cyanoindole.

Preferred compound examples where Z is Formula III are shown below.
5-{3-[4-(2H-benzo[d]1,3-dioxolan-4-ylmethyl)piperidinyl]propoxy}-2-methylbenzothiazole;
5-(3-{4-[(2-bromo-5-methoxyphenyl)methyl]piperidinyl}propoxy)-2-methylbenzothiazole;
5-(3-{4-[(2-bromophenyl)methyl]piperidinyl}propoxy)-2-methylbenzothiazole;
5-(3-{4-[(2-bromo-5-fluorophenyl)methyl]piperidinyl}propoxy)-2-methylbenzothiazole;
5-(3-{4-[(2,-5-difluorophenyl)methyl]piperidinyl}propoxy)-2-methylbenzothiazole;
5-(3-{4-[(3-methoxyphenyl)methyl]piperidinyl}propoxy)-2-methylbenzothiazole;
5-(3-{4-[(2-chlorophenyl)methyl]piperidinyl}propoxy)-2-methylbenzothiazole;
   and
5-(3-{4-[(2,5-dichlorophenyl)methyl]piperidinyl}propoxy)-2-methylbenzothiazole.

The pharmaceutically acceptable acid addition salts of the invention are those in which the counter ion does not contribute significantly to the toxicity or pharmacological activity of the salt and, as such, they are the pharmacological equivalents of the bases of Formula I. They are generally preferred for medical usage. In some instances, they have physical properties which makes them more desirable for pharmaceutical formulation such as solubility, lack of hygroscopicity, compressibility with respect to tablet formation and compatibility with other ingredients with which the substance may be used for pharmaceutical purposes. The salts are routinely made by admixture of a Formula I base with the selected acid, preferably by contact in solution employing an excess of commonly used inert solvents such as water, ether, benzene, methanol, ethanol, ethyl acetate and acetonitrile. They may also be made by metathesis or treatment with an ion exchange resin under conditions in which the anion of one salt of the substance of the Formula I is replaced by another anion under conditions which allow for separation of the desired species such as by precipitation from solution or extraction into a solvent, or elution from or retention on an ion exchange resin. Pharmaceutically acceptable acids for the purposes of salt formation of the substances of Formula I include sulfuric, phosphoric, hydrochloric, hydrobromic, hydroiodic, citric, acetic, benzoic, cinnamic, fumaric, mandelic, phosphoric, nitric, mucic, isethionic, palmitic, heptarioic, and others.

Compounds of Formula I, where Z = II, are most conveniently synthesized by reacting (Reaction 1) intermediate IV (shown as a benzothiazole derivative), where X is a leaving group such as halogen, aryl- or alkyl-sulfonate, or the like, with amine V in a suitable solvent, such as acetonitrile, acetone, DMSO, DMF, and the like, with suitable bases, such as trialkyl amines or sodium, potassium, or cesium carbonate, or the like, under standard alkylation conditions using catalysts such as potassium iodide. In a similar manner in Reaction 2, intermediate IV can be reacted with amine VI to give compounds of Formula I where Z is III. Other methods known to those skilled in the art may also be used.

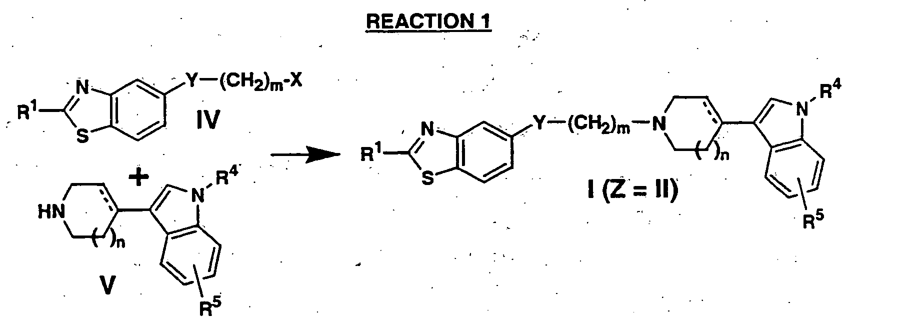

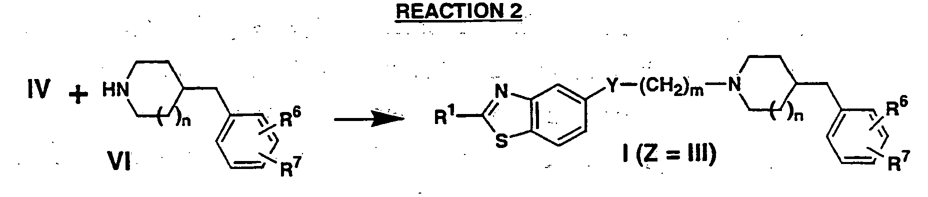

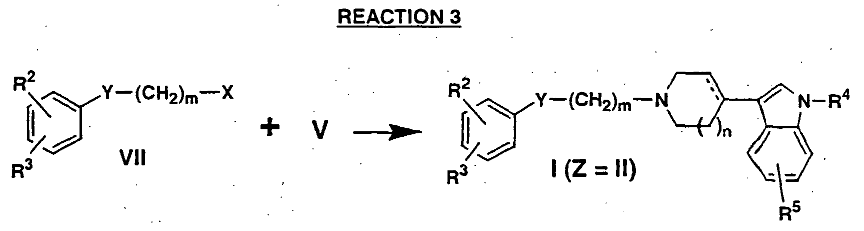

Intermediates of Formulas IV and VII are conveniently made by alkylating (Reactions 4, 5) appropriate benzothiazoles or benzenes with dihaloalkanes in solvents such as acetone, acetonitrile, DMSO, DMF, or the like, and in the presence of suitable bases, such as trialkyl amines or sodium, potassium, or cesium carbonate, or the like, under standard alkylation conditions.

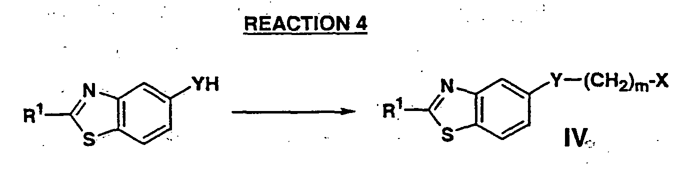

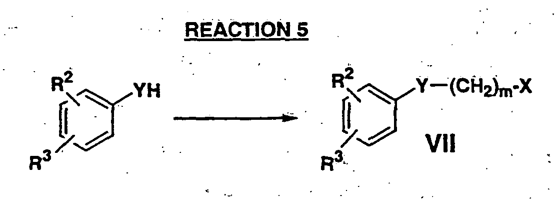

The piperidine intermediates of Formula V (n=1) can be prepared (Reaction 6) by condensing an N-protected-4-piperidone with a substituted indole using catalysts such as pyrrolidine, acetic acid, or the like, in solvents such as ethanol, benzene, THF, or the like, to give the the tetrahydropyridine intermediates (VIII) wherein "PG" represents a nitrogen protecting group. Cleavage of the N-protecting group provides the tetrahydropyridines of Formula Va. Alternatively, the tetrahydropyridine intermediates (VIII) can be reduced using using hydrogen and a suitable catalyst such as platinum, palladium, or ruthenium catalysts, in solvents such as ethanol, ethyl acetate, or the like, to give the piperidine intermediates (IX). The N-protecting group can then be cleaved using methods known to those skilled in the art to give the piperidine intermediates of Formula Vb.

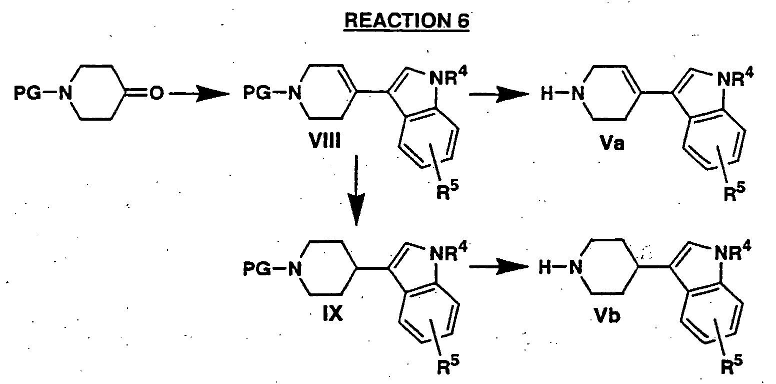

The piperidine intermediates of Formula VI are conveniently prepared by condensation of an N-protected-4-piperidone with reagents such as benzyl phosphonate esters using bases such as NaH, LDA; sodium or potassium alkoxides, or the like, in solvents such as THF, diethyl ether, or the like, to provide the benzylidene intermediate, XI. Subsequent reduction of the benzylidene group using hydrogen and platinum, palladium, or ruthenium catalysts, in solvents such as ethanol, ethyl acetate, or the like, provides the piperidine intermediate, X. The N-protecting group is then cleaved using methods known to those skilled in the art to give the piperidine intermediates of Formula II as depicted in Reaction 7.

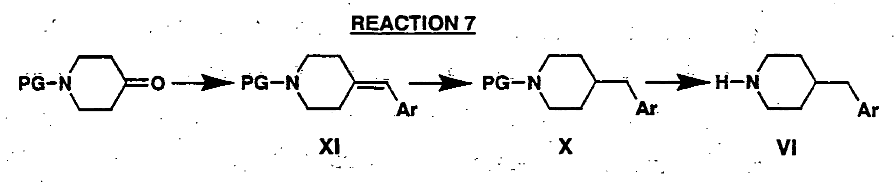

The reactions depicted above and their application are familiar to the practitioner skilled in organic synthesis and modifications of conditions and reagents would be readily understood. The skilled synthetic chemist would know how to adapt these processes for preparation of specific formula I compound including other compounds embraced by this invention but not specifically disclosed. Variations of the methods to produce the same compounds in somewhat different fashion will also be evident to one skilled in the art. To provide greater detail in description, representative synthetic examples are provided infra in the "Specific Embodiments" section.

The compounds of Formula I bind potently to the human 5-HT transporter and inhibit the re-uptake of endogenous serotonin. Selective serotonin reuptake inhibitors (SSRIs) are effective for the treatment of mental depression and have been reported to be useful for treating chronic pain (see: R.W. Fuller, Pharmacologic Modification of Serotonergic Function: Drugs for the Study and Treatment of Psychiatric and Other Disorders," J. Clin. Psychiatry, 47:4 (Suppl.) April 1986, pp. 4-8). Compounds of the present invention are also envisioned to be useful for treating psychosis, acute mania, mild anxiety states or depression with secondary psychotic episodes. The present compounds are also envisioned to be useful in treating obsessive-compulsive disorders, feeding disorders, anxiety disorders and panic disorders.

Like many clinically effective antipsychotic agents, the compounds of Formula I also are antagonists at the human D_{2L} receptor as determined by [³H]-spiperone binding studies using human D_{2L} receptors stably expressed in HEK-293 cells. Clinical studies have demonstrated that selective serotonin reuptake inhibitors (SSRIs) augment the efficacy of traditional neuroleptic antipsychotic agents in improving negative symptoms in schizophrenic parents (Silver, et al., 1998, J. Clin. Psychopharmacol. 18:208; Goff, et al., 1994, Psychopharmacology 117:417). Therefore, the compounds of Formula I possess a unique serotonergic and dopaminergic profile, making the compounds of the present invention useful for treating psychosis, and in particular, the negative symptoms in schizophrenic patients.

Another aspect of the instant invention provides a method for treating a mammal afflicted with psychosis, depression, or chronic pain which comprises administering systemically to said mammal a therapeutically effective amount of a compound of formula I or a pharmaceutically acceptable acid addition salt thereof.

The administration and dosage regimen of compounds of Formula I are considered to be done in the same manner as for the reference compound fluoxetine, cf: Schatzberg, et al., J. Clin. Psychopharmacology 7/6 Suppl. (1987) pp. 4451-4495, and references therein. Although the dosage and dosage regimen must in each case be carefully adjusted, utilizing sound professional judgement and considering the age, weight and condition of the recipient, the route of administration and the nature and gravity of the illness, generally the daily dose will be from about 0.05 to about 10 mg/kg, preferably 0.1 to 2 mg/kg, when administered parenterally and from about 1 to about 50 mg/kg, preferably about 5 to 20 mg/kg, when administered orally. In some instances, a sufficient therapeutic effect can be obtained at lower doses while in others, larger doses will be required. Systemic administration refers to oral, rectal and parenteral (i.e. intramuscular, intravenous and subcutaneous). Generally, it will be found that when a compound of the present invention is administered orally, a larger quantity of the active agent is required to produce the same effect as a similar quantity given parenterally. In accordance with good clinical practice, it is preferred to administer the instant compounds at a concentration level that will produce effective antidepressant effects without causing any harmful or untoward side effects.

The compounds of the present invention may be administered for antipsychotic and antidepressant purposes either as individual therapeutic agents or as mixtures with other therapeutic agents. Therapeutical, they are generally given as pharmaceutical compositions comprised of an antidepressant amount of a compound of Formula I or a pharmaceutically acceptable salt thereof and a pharmaceutically acceptable carrier. Pharmaceutical compositions which provide from about 1 to 500 mg of the active ingredient per unit dose are preferred and are conventionally prepared as tablets, lozenges, capsules, powders, aqueous or oily suspensions, syrups, elixirs, and aqueous solutions.

The nature of the pharmaceutical composition employed will, of course, depend on the desired route of administration. For example, oral compositions may be in the form of tablets or capsules and may contain conventional excipients such as binding agents (e.g. starch) and wetting agents (e.g. sodium lauryl sulfate). Solutions or suspensions of a Formula I compound with conventional pharmaceutical vehicles are employed for parenteral compositions such as an aqueous solution for intravenous injection or an oily suspension for intramuscular injection.

### Description of Specific Embodiments

The compounds which constitute this invention, their methods of preparation and their biologic actions will appear more fully from consideration of the following examples, which are given for the purpose of illustration only and are not to be construed as limiting the invention in sphere or scope. In the following examples, used to illustrate the foregoing synthetic processes, temperatures are expressed in degrees Celsius and melting points are uncorrected. The nuclear magnetic resonance (NMR) spectral characteristics refer to chemical shifts (δ) expressed as parts per million (ppm) versus tetramethylsilane (TMS) as reference standard. The relative area reported for the various shifts in the ¹H NMR spectral data corresponds to the number of hydrogen atoms of a particular functional type in the molecule. The nature of the shifts as to multiplicity is reported as broad singlet (bs), singlet (s), multiplet (m), heptet (hept), quartet (q), triplet (t) or doublet (d). Abbreviations employed are DMSO-d₆ (deuterodimethylsulfoxide), CDCl₃ (deuterochloroform) and are otherwise conventional. The infrared (IR) spectral descriptions include only absorption wave numbers (cm⁻¹).

Analytical thin-layer chromatography (TLC) was performed on 0.25 mm EM silica gel 60 F-254 coated glass plates and preparative flash chromatography was performed on EM silica gel (36-62 µm). The solvent systems used are reported where appropriate. All reaction, extraction and chromatography solvents were reagent grade and used without further purification except tetrahydrofuran (THF) which was distilled from sodium/benzophenone ketyl. All non-aqueous reactions were carried out in flame-dried glassware under a nitrogen atmosphere.

### Synthesis of Intermediates of Formula IV

### Example 1: 2-methyl-5-(3-chloropropoxyl)-benzothiazole

A mixture of 2-methyl-5-benzothiazolol (8 g, 48.4 mmol), 1-bromo-3-chloropropane (23 g, 146 mmol), and potassium carbonate (33.6 g, 240 mmol) in 50 ml of acetonitrile was stirred at 60 °C for 18 hr. The mixture was cooled, evaporated under reduced pressure, diluted with water and ethyl acetate. The organic phase was separated, dried over sodium sulfate, and concentrated under vacuum to give 2-methyl-5-(3-chloropropoxyl)-benzothiazole (IV) as a tan crystalline solid (10 g, 85%, mp 63-65 °C, MS (Cl) m+1=242).

In a similar manner, phenols and thiophenols can be reacted with a dihaloalkane to produce the corresponding benzene intermediates of Formula VII.

### Synthesis of Intermediates of Formula VI

### Example 2: 4-(2-bromobenzyl)piperidine

A solution of dimethyl 2-bromobenzylphosphonate (45.66 g, 148.9 mmol) in THF was added slowly to a mixture of NaH (7.14 g of a 60% mineral oil dispersion, 178.5 mmol) in THF (200 ml) and the mixture was stirred for 1 hr. A solution of 1-(tert-butoxycarbonyl)-4-piperidinone (29.67 g, 148.9 mmol) in THF was added dropwise and the mixture was heated to reflux for 1.5 hr. The mixture was cooled and quenched with brine. The mixture was diluted with ethyl acetate, washed with water, and dried with brine. The organic layer was concentrated in vacuo to an oil. The oil was dissolved in acetonitrile and extracted with hexane. The acetonitrile layer was concentrated in vacuo to give 1-(tert-butoxycarbonyl)-4-[(2-bromophenyl)methylene]piperidine (XI) as an oil that solidified upon standing (48.3 g, 97%).

A solution of 1-(tert-butoxycarbonyl)-4-[(2-bromophenyl)methylene]-piperidine (XI, 8 g, 22.7 mmole) in ethyl acetate (75 ml) and ethanol (75 ml) was shaken with PtO₂ (0.75 g) and hydrogen (60 psi) for 15 min. Two further batches of 1-(tert-butoxycarbonyl)-4-[(2-bromophenyl)-methylene]-piperidine (8 g each, 24 g total) were similarly reduced and the mixtures were filtered. The filtrates were combined and concentrated in vacuo. The residue (X) was dissolved in dioxane (200 ml) and 3N HCl (100 ml) and stirred for 18 hr. The solution was concentrated in vacuo and the residue was made basic with 50% sodium hydroxide. The mixture was extracted with CH₂Cl₂. The extracts were dried over Na₂SO₄ and concentrated in vacuo to give a yellow oil that was purified by short path vacuum distillation to give 4-(2-bromobenzyl)piperidine (VI) as a oil (15 g, 86.6%). The oil converted to the fumarate salt using fumaric acid (6.85 g) in 2-propanol to give 4-(2-bromobenzyl)piperidine fumarate as a white solid (15.8 g, 62.6% overall, mp: 164-165 °C).

Also prepared by this general method were:
4-(2-bromo-5-fluorobenzyl)piperidine;
4-(2-bromo-5-methoxybenzyl)piperidine;
4-(2,5-dichlorobenzyl)piperidine; and
4-(2-chlorobenzyl)piperidine.

### Synthesis of Intermediates of Formula V

### Example 3: 3-(4-Piperidinyl)-5-cyanoindole (V)

### A. 3-[1-(t-butoxycarbonyl)-1,2,3,6-tetrahydro-4-pyridinyl]-5-cyanoindole (VIII)

A stirred solution of 5-cyanoindole (10.0 g, 70.3 mmol), t-butyl-4-oxo-1-piperidinecarboxylate (15.4 g, 77.3 mmol), and pyrrolidine (14.7 mL, 175.7 mmol) in ethanol (200 mL) was heated at reflux for 20 hours then cooled to 0°C. The resulting precipitate was collected by vacuum filtration and rinsed with cold ethanol and hexanes to afford the product as a white solid (15.4 g, 68%).

### B. 3-[1-(t-butoxycarbonyl)-4-piperidinyl]-5-cyanoindole (IX)

A solution of 3-[1-(t-butoxycarbonyl)-1,2,3,6-tetrahydro-pyridin-4-yl]-5-cyanoindole (15.3 g, 47.3 mmol) in ethanol was treated with 10% palladium on carbon (2.38 g). The mixture was hydrogenated on a Parr shaker at 50 psi for 18 hours and then filtered through celite to remove inorganics. Evaporation gave the desired product as an off white solid (15.2 g, 98%). ¹H-NMR δ (CDCl₃) 8.33 (br s, 1 H), 7.99 (s, 1 H), 7.42 (s, 2 H), 7.07 (s, 1 H), 4.22 (m, 2 H), 2.82 (m, 3 H), 1.98 (br d, 2 H), 1.66 (m, 2 H), 1.48 (s, 9 H).

### C. 3-(4-piperidinyl)-5-cyanoindole (Vb)

A stirred solution of 3-[1-(t-butoxycarbonyl)-4-piperidyl]-5-cyanoindole (8.4 g, 25.8 mmol) in methanol (260 mL) was treated with 4N hydrochloric acid in dioxane (64 mL, 256 mmol). After stirring at ambient temperature for 2 hours, the solution was concentrated. The residue was neutralized with 1 N NaOH, extracted with ethyl acetate, dried, and concentrated to afford the product as a white solid (5.29 g, 91%). ¹H-NMR δ (CDCl₃) 8.59 (br s, 1 H), 8.00 (s, 1 H), 7.40 (s, 2 H), 7.09 (s, 1 H), 3.23 (br d, 1 H), 2.93 (m, 1 H), 2.81 (br t, 2 H), 2.01 (m, 2 H), 1.66 (m, 4 H).

By using VIII instead of IX in the hydrolysis reaction (C), the corresponding tetrahydropyridine intermediate Va is produced.

By starting with an appropriate VIII intermediate and using the procedures of Example 3, selected Va and/or Vb intermediates are readily prepared. A number of descriptive preparations of assorted Formula VIII intermediates follow.

### A. 3-[1-(t-butoxycarbonyl)-1,2,3,6-tetrahydro-4-pyridinyl]-4-fluoroindole

A stirred solution of 4-fluoroindole (400 mg, 3.0 mmol), t-butyl-4-oxo-1-piperidinecarboxylate (650 mg, 3.3 mmol), and pyrrolidine (0.62 mL, 7.4 mmol) in ethanol (10 mL) was heated at reflux for 16 hours. Solvent was evaporated and the residue subjected to chromatography on silica gel with 20% ethyl acetate/ hexanes to afford a yellow solid (250 mg, 27%).

### B. 3-[1-(t-butoxycarbonyl)-1,2,3,6-tetrahydro-4-pyridinyl]-7-fluoroindole

A stirred solution of 7-fluoroindole (400 mg, 3.0 mmol), t-butyl-4-oxo-1-piperidinecarboxylate (650 mg, 3.3 mmol), and pyrrolidine (0.62 mL, 7.4 mmol) in ethanol (10 mL) was heated at reflux for 16 hours. Solvent was evaporated and the residue subjected to chromatography on silica gel with 20% ethyl acetate/ hexanes to afford a yellow solid (620 mg, 66%).

### C. 3-[1-(t-butoxycarbonyl)-1,2,3,6-tetrahydro-4-pyridinyl]-5-chloroindole

A stirred solution of 5-chloroindole (455 mg, 3.0 mmol), t-butyl-4-oxo-1-piperidinecarboxylate (650 mg, 3.3 mmol), and pyrrolidine (0.62 mL, 7.4 mmol) in ethanol (10 mL) was heated at reflux for 16 hours. Solvent was evaporated and the residue subjected to chromatography on silica gel with 20% ethyl acetate/ hexanes to afford a yellow solid (752 mg, 75%).

### D. 3-[1-(t-butoxycarbonyl)-1,2,3,6-tetrahydro-4-pyridinyl]-6-chloroindole

A stirred solution of 6-chloroindole (455 mg, 3.0 mmol), t-butyl-4-oxo-1-piperidinecarboxylate (650 mg, 3.3 mmol), and pyrrolidine (0.62 mL, 7.4 mmol) in ethanol (10 mL) was heated at reflux for 16 hours. Solvent was evaporated and the residue was recrystallized from ethyl acetate and hexanes to afford a yellow solid (721 mg, 72%).

### E. 3-[1-(t-butoxycarbonyl)-1,2,3,6-tetrahydro-4-pyridinyl]-6-bromoindole

A stirred solution of 6-bromoindole (390 mg, 2.0 mmol), t-butyl-4-oxo-1-piperidinecarboxylate (438 mg, 2.2 mmol), and pyrrolidine (0.42 mL, 5.0 mmol) in ethanol (10 mL) was heated at reflux for 16 hours. Solvent was evaporated and the residue subjected to chromatography on silica gel with 20% ethyl acetate/ hexanes to afford a yellow solid (354 mg, 47%).

### F. 3-[1-(t-butoxycarbonyl)-1,2,3,6-tetrahydro-4-pyridinyl]-7-bromoindole

A stirred solution of 7-bromoindole (390 mg, 2.0 mmol), t-butyl-4-oxo-1-piperidinecarboxylate (438 mg, 2.2 mmol), and pyrrolidine, (0.42 mL, 5.0 mmol) in ethanol (10 mL) was heated at reflux for 16 hours. Solvent was evaporated and the residue subjected to chromatography on silica gel with 20% ethyl acetate/ hexanes to afford a yellow solid (215 mg, 29%).

### Synthesis of Compounds of Formula I

### A. Benzene Derivatives

### Example 4: 3-[1-(3-phenoxypropyl)-4-piperidinyl]-5-cyanoindole

A stirred solution of 3-(4-piperidinyl)-5-cyanoindole (0.71 g, 2.2 mmol), 3-phenoxypropyl bromide (0.52 g, 2.4 mmol), and triethylamine (0.6 ml, 4.4 mmol) in methanol (10 mL) was heated at reflux for 18 hours. The solution was concentrated *in vacuo*. Purification by flash chromatography yielded the product (0.35 g, 44%). ¹H-NMR δ (CD₃OD) 8.11 (s, 1 H), 7.49 (d, 1 H), 7.40 (d, 1 H), 7.28 (m, 3 H), 6.94 (m, 3 H), 4.12 (t, 2 H), 3.59 (br d, 2 H), 3.26-3.18 (m, 3 H), 3.04 (br t, 2 H), 2.25 (m, 4 H), 2.02 (br q, 2 H).

The following compounds were prepared essentially as described for the previous preparation:

### Example 5: 3-{1-[3-(2-fluorophenoxy)propyl]-4-piperidinyl}-5-cyanoindole

¹H-NMR δ (CD₃OD) 8.10 (s, 1 H), 7.49 (d, 1 H), 7.37 (s, 1 H), 7.26 (s, 1 H), 7.12 (m, 3 H), 6.93 (m, 1 H), 4.19 (t, 2 H), 3.46 (br d, 2 H), 3.18 (m, 3 H), 2.80 (br t, 2 H), 2.20 (m, 4 H), 2.00 (br q, 2 H).

### Example 6: 3-{1-[3-(4-fluorophenoxy)propyl]-4-piperidinyl}-5-cyanoindole

¹H-NMR δ (CD₃OD) 8.10 (s, 1 H), 7.48 (d, 1 H), 7.39 (d, 1 H), 7.27 (s, 1 H), 7.01 (m, 2 H), 6.92 (m, 2 H), 4.06 (t, 2 H), 3.46 (br d, 2 H), 3.07 (m, 3 H), 2.81 (br t, 2 H), 2.19 (m, 4 H), 1.98 (br q, 2 H).

### Example 7: 3-{1-[3-(2-methoxyphenoxy)propyl]-4-piperidinyl}-5-cyanoindole

¹H-NMR δ (CD₃OD) 8.10 (s, 1 H), 7.49 (d, 1 H), 7.38 (d, 1 H), 7.28 (s, 1 H), 6.95 (m, 4 H), 4.13 (t, 2 H), 3.83 (s, 3 H), 3.52 (br d, 2 H), 3.13 (m, 3 H), 2.85 (br t, 2 H), 2.22 (m, 4 H), 1.99 (br q, 2 H).

### Example 8: 3-{1-[3-(3-methoxyphenoxy)propyl]-4-piperidinyl}-5-cyanoindole

¹H-NMR δ (CD₃OD) 8.08 (s, 1 H), 7.47 (d, 1 H), 7.36 (d, 1 H), 7.22 (s, 1 H), 7.15 (t, 1 H), 6.50 (m, 3 H), 4.03 (t, 2 H), 3.76 (s, 3 H), 3.18 (br d, 2 H), 2.89 (m, 1 H), 2.69 (m, 2 H), 2.33 (br t, 2 H), 2.05 (m, 4 H), 1.88 (br q, 2 H).

### Example 9: 3-{1-[3-(4-methoxyphenoxy)propyl]-4-piperidinyl}-5-cyanoindole

¹H-NMR δ (CD₃OD) 8.10 (s, 1 H), 7.49 (d, 1 H), 7.38 (d, 1 H), 7.28 (s, 1 H), 6.86 (m, 4 H), 4.05 (t, 2 H), 3.74 (s, 3 H), 3.52 (br d, 2 H), 3.13 (m, 3 H), 2.90 (br t, 2 H), 2.18 (m, 4 H), 1.98 (br q, 2 H).

### Example 10: 3-{1-[3-(3,4-dimethoxyphenoxy)propyl]-4-piperidinyl}-5-cyanoindole

¹H-NMR δ (CD₃OD) 8.09 (s, 1 H), 7.48 (d, 1 H), 7.38 (d, 1 H), 7.25 (s, 1 H), 6.85 (d, 1 H), 6.59 (s, 1 H), 6.46 (dd, 1 H), 4.02 (t, 2 H), 3.81 (s, 3 H), 3.78 (s, 3 H), 3.34 (br d, 2H), 3.00 (m, 1 H), 2.89 (t, 2 H), 2.59 (br t, 2 H), 2.10 (m, 4 H), 1.92 (br q, 2 H).

### B. Benzothiazole Derivatives

### Example 11: 5-{3-[4-(4-fluoroindol-3-yl)-1,2,5,6-tetrahydropyrid-1-yl]propoxy}-2-methylbenzothiazole

To a solution of 3-[1-(t-butoxycarbonyl)-1,2,3,6-tetrahydro-4-pyridinyl]-4-fluoroindole (47 mg, 0.15 mmol) in dichloromethane (2 mL) was added hydrochloric acid (1 mL, 4N in dioxane, 4 mmol). This mixture was stirred for 2 hours and was then evaporated to dryness. To this was added methanol (2 mL) and triethylamine (0.1 mL, 0.73 mmol) and this solution was transferred to a glass vial containing 1-(3-(2-methyl-5-benzothiazoxy)-bromopropane (43 mg, 0.15 mmol). The reaction was sealed and heated at 65 °C for 2 days. The reaction was filtered and purified on a Shimadzu preparative HPLC system with the following conditions:

Reverse Phase Column 30x75 mm; start %B = 20; final %B = 100; gradient time = 12 min; flow rate = 40 mL/min; wavelength = 220 nm.

The product was evaporated to dryness and the TFA salt was obtained as an orange oil (30 mg, 38%).

¹H-NMR δ (CDCl₃) 2.37 (m, 2H), 2.79 (bd, *J* = 16 Hz, 1H), 2.92 (s, 3H), 3.01 (m, 1H), 3.28 (m, 1H), 3.44 (m, 2H), 3.64 (bd, *J* =16.2 Hz, 1H), 3.76 (m, 1H), 4.15(t, *J* = 5.5 Hz, 2H), 4.23 (bd, *J* = 16.1 Hz, 1H), 5.83 (bs, 1H), 5.98 (s, 1H), 6.78 (dd, *J* = 11.5, 7.7 Hz, 1H), 7.03-7.18 (m, 3H), 7.57 (s, 1H), 7.69 (d, *J* = 8.8 Hz, 1H), 8.95 (s, 1H).

### Examole 12: 5-{3-[4-(5-cyanoindol-3-yl)-1,2,5,6-tetrahydropyrid-1-yl]propoxy}-2-methylbenzothiazole

A mixture of 2-methyl-5-(3-chloropropoxyl)-benzothiazole (3 g, 12.4 mmol), 5-cyano-3-(1,2,3,6-tetrahydropyridin-4-yl)-1H-indole (3 g, 13.4 mmol) and a catalytic amount of potassium iodide in 50 ml of acetonitrile was stirred at 80 °C overnight. The reaction mixture was cooled, diluted with water, ammonium hydroxide and ethyl acetate. The two phases were separated and the aqueous phase was extracted with ethyl acetate twice. The organic phases were combined, washed with brined, dried over sodium sulfate and then concentrated under reduced pressure to a solid. This residual solid was subjected to a silica gel chromatography, eluting with 7% methanol in dichloromethane. Fractions shown to contain product were combined and concentrated to provide 2 g of the title compound in 35% yield as free base. The resulting free base was dissolved in methanol/dichloromethane and precipitated with excess HCl in ether. Recrystalization from ethanol give 1.8 g of the title compound as a light yellow solid. mp. 263-65 °C. MS(CI) m+1=429. EA (C₂₅H₂₄N₄ OS·1.7HCl): Theory: C, 61.21%; H, 5.28%; N, 11.42%. Found: C, 61.10%; H, 5.43%; N, 11.39%.

Also prepared by this general method from the appropriate starting materials were:
Example 13: 5-{4-[4-(4-fluoroindol-3-yl)1,2,5,6-tetrahydropyrid-1-yl]butoxy}-2-methylbenzothiazole;
Example 14: 5-{5-[4-(4-fluoroindol-3-yl)-1,2,5,6-tetrahydropyrid-1-yl]pentoxy}-2-methylbenzothiazole;
Example 15: 5-{4-[4-(5-fluoroindol-3-yl)1,2,5,6-tetrahydropyrid-1-yl]butoxy}-2-methylbenzothiazole;
Example 16: 5-{5-[4-(5-fluoroindol-3-yl)-1,2,5,6-tetrahydropyrid-1-yl]pentoxy}-2-methylbenzothiazole;
Example 17: 5-{3-[4-(7-fluoroindol-3-yl)-1,2,5,6-tetrahydropyrid-1-yl]propoxy}-2-methylbenzothiazole;
Example 18: 5-{4-[4-(7-fluoroindol-3-yl)1,2,5,6-tetrahydropyrid-1-yl]butoxy}-2-methylbenzothiazole;
Example 19: 5-{5-[4-(7-fluoroindol-3-yl)-1,2,5,6-tetrahydropyrid-1-yl]pentoxy}-2-methylbenzothiazole;
Example 20: 5-{4-[4-(5-chloroindol-3-yl)1,2,5,6-tetrahydropyrid-1-yl]butoxy}-2-methylbenzothiazole;
Example 21: 5-{5-[4-(5-chloroindol-3-yl)-1,2,5,6-tetrahydropyrid-1-yl]pentoxy}-2-methylbenzothiazole;
Example 22: 5-{3-[4-(6-chloroindol-3-yl)-1,2,5,6-tetrahydropyrid-1-yl]propoxy}-2-methylbenzothiazole;
Example 23: 5-{4-[4-(6-chloroindol-3-yl)1,2,5,6-tetrahydropyrid-1-yl]butoxy}-2-methylbenzothiazole;
Example 24: 5-{5-[4-(6-chloroindol-3-yl)-1,2,5,6-tetrahydropyrid-1-yl]pentoxy}-2-methylbenzothiazole;
Example 25: 5-{3-[4-(6-bromoindol-3-yl)-1,2,5,6-tetrahydropyrid-1-yl]propoxy}-2-methylbenzothiazole;
Example 26: 5-{4-[4-(6-bromoindol-3-yl)1,2,5,6-tetrahydropyrid-1-yl]butoxy}-2-methylbenzothiazole;
Example 27: 5-{5-[4-(6-bromoindol-3-yl)-1,2,5,6-tetrahydropyrid-1-yl]pentoxy}-2-methylbenzothiazole;
Example 28: 5-{3-[4-(7-bromoindol-3-yl)-1,2,5,6-tetrahydropyrid-1-yl]propoxy}-2-methylbenzothiazole;
Example 29: 5-{4-[4-(7-bromoindol-3-yl)1,2,5,6-tetrahydropyrid-1-yl]butoxy}-2-methylbenzothiazole;
Example 30: 5-{5-[4-(7-bromoindol-3-yl)-1,2,5,6-tetrahydropyrid-1-yl]pentoxy}-2-methylbenzothiazole;
Example 31: 5-{3-[4-(5-methoxyindol-3-yl)-1,2,5,6-tetrahydropyrid-1-yl]propoxy}-2-methylbenzothiazole;
Example 32: 5-{4-[4-(5-methoxyindol-3-yl)1,2,5,6-tetrahydropyrid-1-yl]butoxy}-2-methylbenzothiazole;
Example 33: 5-{5-[4-(5-methoxyindol-3-yl)-1,2,5,6-tetrahydropyrid-1-yl]pentoxy}-2-methylbenzothiazole;
Example 34: 5-{3-[4-(5-fluoroindol-3-yl)-1,2,5,6-tetrahydropyrid-1-yl]propoxy}-2-methylbenzothiazole;
Example 35: 5-{3-[4-(5-fluoroindol-3-yl)piperidinyl]propoxy}-2-methylbenzothiazole;
Example 36: 5-{3-[4-(5-bromoindol-3-yl)-1,2,5,6-tetrahydropyrid-1-yl]propoxy}-2-methylbenzothiazole;
Example 37: 5-{3-[4-(5-cyanoindol-3-yl)piperidinyl]propoxy}-2-methylbenzothiazole;
Example 38: 5-{4-[4-(5-cyanoindol-3-yl)-1,2,5,6-tetrahydropyrid-1-yl]butoxy}-2-methylbenzothiazole;
Example 39: 5-{4-[4-(5-cyanoindol-3-yl)piperidinyl]butoxy}-2-methylbenzothiazole; and
Example 40: 2-methyl-5-[3-[4-(2-bromo-5-fluorobenzyl)-piperidinyl]-propoxyl]-benzothiazole.

The title compound was prepared in similar fashion from 2-methyl-5-(3-chloropropoxyl)-benzothiazole and 2-bromo-5-fluorobenzyl)-piperidine. The resulting free base (40% yield)was dissolved in ethyl acetate and precipitated with one equivalent of HCl in ether. mp. 217-20 °C. MS(Cl) m+1=477. EA (C₂₃H₂₆BrFN₂OS·HCl·H2O): Theory: C, 51.94%; H, 5.50%; N, 5.27%. Found: C, 52.05%; H, 5.34%; N, 5.23%.

Also prepared by this general method from the appropriate starting materials were:
Example 41: 5-{3-[4-(2H-benzo[d]1,3-dioxolan-4-ylmethyl)piperidinyl]propoxy}-2-methylbenzothiazole;
Example 42: 5-(3-{4-[(2-bromo-5-methoxyphenyl)methyl]piperidinyl}propoxy)-2-methylbenzothiazole;
Example 43: 5-(3-{4-[(2-bromophenyl)methyl]piperidinyl}propoxy)-2-methylbenzothiazole;
Example 44: 5-(3-{4-[(2-bromo-5-fluorophenyl)methyl]piperidinyl}propoxy)-2-methylbenzothiazole;
Example 45: 5-(3-{4-[(2,-5-difluorophenyl)methyl]piperidinyl}propoxy)-2-methylbenzothiazole;
Example 46: 5-(3-{4-[(3-methoxyphenyl)methyl]piperidinyl}propoxy)-2-methylbenzothiazole;
Examale 47: 5-(3-{4-[(2-chlorophenyl)methyl]piperidinyl}propoxy)-2-methylbenzothiazole; and
Example 48: 5-(3-{4-[(2,5-dichlorophenyl)methyl]piperidinyl}propoxy)-2-methylbenzothiazole.

### Dopamine Binding Protocol

HEK-293 cells that stably express recombinant human dopamine D_{2L} receptors (HEK-D_{2L} cells) were grown at 37 °C in 5% CO₂ as a monolayer in medium consisting of EMEM supplemented with 10% fetal bovine serum and G418 sulfate (500 µg/ml). To prepare membranes for radioligand binding experiments, cells were rinsed twice with phosphate-buffered saline (138 mM NaCl, 4.1 mM KCl, 5.1 mM Na₂PO₄, 1.5 mM KH₂PO₂ 11.1 mM glucose, pH 7.4), and incubated for 5-10 min. at 4 °C in hypotonic lysis buffer consisting of 10 mM Tris (pH 7.4) and 5 mM EDTA. Cells were transferred from plates to polypropylene tubes (16 x 100 mm), homogenized and centrifuged at 32,000 x g for 20 min. Following centrifugation, pellets were resuspended by homogenization in buffer consisting of 50 mM Tris (pH 7.7 at 25 °C) and 1 mM EDTA. Homogenates were stored at -80 °C until needed. On the day of an experiment, hornogeriates were thawed then centrifuged at 32,000 x g for 20 min. Folloinimg centrifugation, supernatants were discarded and pellets were resuspended in assay buffer consisting of 50 mM Tris (pH 7.4 at 25 °C), 1 mM EDTA and 6 mM MgCl₂. Membrane homogenates (∼5 µg) were incubated with 150 pM [³H]-spiperone (Amersham Life Science) and increasing concentrations of test compounds for 1.5 hours at 22 °C in a total volurne of 400 µl. Reactions were stopped by addition of ice-cold assay buffer and filtration over glass fiber filters (Whatman GFB, pre-soaked in 0.05% polyethylenimine) using a microtitre format Brandel cell harvester. Filters were washed with 3 ml of ice-cold assay buffer. Non-specific binding was defined with 2 µM (+)butaclamol. Ki values were calculated using the method of Cheng and Ptusoff (1973). Protein concentrations were determined by the method of Bradford (1976) with BSA as a standard.

Test data IC₅₀ values lower than 250 nM are considered to reflect affinity for dopamine D_{2L} receptors. Compounds with IC₅₀ values lower than 100 nM comprise preferred compounds.

### Serotonin Transporter Binding Assay

HEK-293 cells that stably express human serotonin transporters (HEK-hSERT cells) were grown at 37 °C in 5% CO₂ as a monolayer in medium consisting of EMEM supplemented with 10% fetal bovine serum and G418 sulfate (500 µg/ml). To prepare membranes for radiotigand binding experiments, cells were rinsed twice with phosphate-buffered saline (138 mM NaCl, 4.1 mM KCI, 5.1 mM Na₂PO₄, 1.5 mM KH₂O₄, 11.1 mM glucose, pH 7.4). Cells-were transferred from plates to polypropylene tubes (16 x 100 mm), centrifuged at 1,200 x g for 5 min and were frozen at -80 °C until assay. Following centrifugation, pellets were resuspended by homogenization in buffer consisting of 50 mM Tris (pH 7.7 at 25 °C), 120 mM NaCl and 5 mM KCl and then centrifuged at 32,000 x g for 10 min. Following centrifugation, supernatants were discarded and pellets were resuspended in buffer consisting of 50 mM Tris (pH 7.4 at 25 °C), 150 mM NaCl and 5 mM KCl. Membrane homogenates (20.0 µl/plate) were incubated with 1 nM [³H]-citalopram (specific activity = 85 Ci/mmol) and increasing concentrations of test compounds for 1 hour at 25 °C. in a total volume of 250 µl. The assay buffer consisted of 50 mM Tris (pH 7.4 at 25 °C), 120 mM NaCl and 5 mM KCI (pH 7.4 with conc. HCl): Plates were incubated for 1 hour at 25 °C, then filtered through 0.5% PEI treated Whatman GF/B filters using a Brandel cell harvester. Filters were washed three times with 3 ml of ice-cold tris wash buffer: Non-specific binding was defined with 10 µM fluoxetine. Amount of radioligand bound in the presence and absence of competitor was analyzed by plotting (-)log drug concentration versus the amount of radioligand specifically bound. The midpoint of the displacement curve (IC₅₀, nM), signifies the potency. Kᵢ values were calculated using the method of Cheng and Prusoff (1973).

Substances which inhibit the re-uptake of serotonin are recognized to be effective antidepressants (Selective Serotonin Reuptake Inhibitors. Edited by JP Feighner and WF Boyer, Chichester, England. John Wiley & Sons, 1991, pp 89-108). Test data IC₅₀ values lower than 250 nM are considered to reflect activity as an inhibitor of serotonin re-uptake. Compounds with IC₅₀ values lower than 100 nM comprise preferred compounds.

The following compounds of Formula I inhibit the re-uptake of serotonin with Ki lower than 100 nM, and are dopamine D_{2L} ligands with Ki lower than 100 nM:
5-{3-[4-(5-fluoroindol-3-yl)-1,2,5,6-tetrahydropyrid-1-yl]propoxy}-2-methylbenzothiazole;
5-{3-[4-(5-cyanoindol-3-yl)-1,2,5,6-tetrahydropyrid-1-yl]propoxy}-2-methylbenzothiazole;
5-{3-[4-(5-fluoroindol-3-yl)piperidinyl]propoxy}-2-methylbenzothiazole;
5-{3-[4-(5-cyanoindol-3-yl)piperidinyl]propoxy}-2-methylbenzothiazole;
5-{4-[4-(5-cyanoindol-3-yl)-1,2,5,6-tetrahydropyrid-1-yl]butoxy}-2-methylbenzothiazole;
5-{4-[4-(5-cyanoindol-3-yl)piperidinyl]butoxy}-2-methylbenzothiazole;
5-{3-[4-(5-bromoindol-3-yl)-1,2,5,6-tetrahydropyrid-1-yl]propoxy}-2-methylbenzothiazole;
5-{3-[4-(2H-benzo[d]1,3-dioxolan-4-ylmethyl)piperidinyl]propoxy}-2-methylbenzothiazole;
5-(3-{4-[(2-bromo-5-methoxyphenyl)methyl]piperidinyl}propoxy)-2-methylbenzothiazole;
5-(3-{4-[(2-bromophenyl)methyl]piperidinyl}propoxy)-2-methylbenzothiazole;
5-(3-{4-[(2-bromo-5-fluorophenyl)methyl]piperidinyl}propoxy)-2-methylbenzothiazole;
5-(3-{4-[(2,-5-difluorophenyl)methyl]piperidinyl}propoxy)-2-methylbenzothiazole;
5-(3-{4-[(3-methoxyphenyl)methyl]piperidinyl}propoxy)-2-methylbenzothiazole;
5-(3-{4-[(2-chlorophenyl)methyl]piperidinyl}propoxy)-2-methylbenzothiazole;
5-(3-{4-[(2,5-dichlorophenyl)methyl]piperidinyl}propoxy)-2-methylbenzothiazole;
3-[1-(3-phenoxypropyl)-4-piperidinyl]-5-cyanoindole;
3-{1-[3-(4-fluorophenoxy)propyl]-4-piperidinyl}-5-cyanoindole;
3-{1-[3-(2-fluorophenoxy)propyl]-4-piperidinyl}-5-cyanoindole;
3-{1-[3-(2-methoxyphenoxy)propyl]-4-piperidinyl}-5-cyanoindole;
3-{1-[3-(3-methoxyphenoxy)propyl]-4-piperidinyl}-5-cyanoindole;
3-{1-[3-(4-methoxyphenoxy)propyl]-4-piperidinyl}-5-cyanoindole;
3-{1-[3-(3,4-dimethoxyphenoxy)propyl]-4-piperidinyl}-5-cyanoindole;
5-{3-[4-(4-ftuoroindol-3-yl)-1,2,5,6-tetrahydropyrid-1-yl]propoxy}-2-methylbenzothiazole;
5-{3-[4-(7-fluoroindol-3-yl)-1,2,5,6-tetrahydropyrid-1-yl]propoxy}-2-methylbenzothiazole;
5-{3-[4-(6-chloroindol-3-yl)-1,2,5,6-tetrahydropyrid-1-yl]propoxy}-2-methylbenzothiazole;
5-{3-[4-(6-bromoindol-3-yl)-1,2,5,6-tetrahydropyrid-1-yl]propoxy}-2-methylbenzothiazole;
5-{3-[4-(7-bromoindol-3-yl)-1,2,5,6-tetrahydropyrid-1-yl]propoxy}-2-methylbenzothiazole;
5-{3-[4-(5-methoxyindol-3-yl)-1,2,5,6-tetrahydropyrid-1-yl]propoxy}-2-methylbenzothiazole;
5-{4-[4-(4-fluoroindol-3-yl)1,2,5,6-tetrahydropyrid-1-yl]butoxy}-2-methylbenzothiazole;
5-{4-[4-(5-fluoroindol-3-yl)1,2,5,6-tetrahydropyrid-1-yl]butoxy}-2-methylbenzothiazole;
5-{4-[4-(7-fluoroindol-3-yl)1,2,5,6-tetrahydropyrid-1-yl]butoxy}-2-methylbenzothiazole;
5-{4-[4-(5-chloroindol-3-yl)1,2,5,6-tetrahydropyrid-1-yl]butoxy}-2-methylbenzothiazole;
5-{4-[4-(6-chloroindol-3-yl)1,2,5,6-tetrahydropyrid-1-yl]butoxy}-2-methylbenzothiazole;
5-{4-[4-(6-bromoindol-3-yl)1,2,5,6-tetrahydropyrid-1-yl]butoxy}-2-methylbenzothiazole;
5-{4-[4-(7-bromoindol-3-yl)1,2,5,6-tetrahydropyrid-1-yl]butoxy}-2-methylbenzothiazole;
5-{5-[4-(4-fluoroindol-3-yl)-1,2,5,6-tetrahydropyrid-1-yl]pentoxy}-2-methylbenzothiazole;
5-{5-[4-(5-fluoroindol-3-yl)-1,2,5,6-tetrahydropyrid-1-yl]pentoxy}-2-methylbenzothiazole;
5-{5-[4-(7-fluoroindol-3-yl)-1,2,5,6-tetrahydropyrid-1-yl]pentoxy}-2-methylbenzothiazole;
5-{5-[4-(5-chloroindol-3-yl)-1,2,5,6-tetrahydropyrid-1-yl]pentoxy}-2-methylbenzothiazole;
5-{5-[4-(6-chloroindol-3-yl)-1,2,5,6-tetrahydropyrid-1-yl]pentoxy}-2-methylbenzothiazole;
5-{5-[4-(6-bromoindol-3-yl)-1,2,5,6-tetrahydropyrid-1-yl]pentoxy}-2-methylbenzothiazole;
5-{5-[4-(7-bromoindol-3-yl)-1,2,5,6-tetrahydropyrid-1-yl]pentoxy}-2-methylbenzothiazole;
5-{5-[4-(5-methoxyindol-3-yl)-1,2,5,6-tetrahydropyrid-1-yl]pentoxy}-2-methylbenzothiazole.

## Claims

1. A compound of Formula I and its pharmaceutically acceptable salts 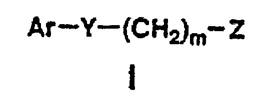 wherein:
Ar is 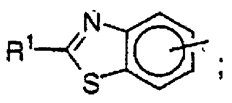
Y is selected from oxygen and sulfur;
Z is II: 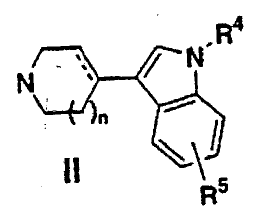
R¹ and R⁴ are independently selected from hydrogen and C₁₋₄ alkyl;
R⁵ is selected from hydrogen, halogen, C₁₋₄ alkoxy and cyano;
m is an integer from 2 to 6;
n is the integer 1; and
a dotted line represents an optional double bond.

2. A compound of claim 1 wherein m is 3 and Y is oxygen.

3. A compound of claim 1 selected from:
5-{3-[4-(5-fluoroindol-3-yl)-1,2,5,6-tetrahydropyrid-1-yl]propoxy}-2-methylbenzothiazole;
5-{3-[4-(5-cyanoindol-3-yl)-1,2,5,6-tetrahydropyrid-1-yl]propoxy}-2-methylbenzothiazole;
5-{3-[4-(5-fluoroindol-3-yl)piperidinyl]propoxy}-2-methylbenzothiazole;
5-{3-[4-(5-cyanoindol-3-yl)piperidinyl]propoxy}-2-methylbenzothiazole;
5-{4-[4-(5-cyanoindol-3-yl)-1,2,5,6-tetrahydropyrid-1-yl]butoxy}-2-methylbenzothiazole;
5-{4-[4-(5-cyanoindol-3-yl)piperidinyl]butoxy}-2-methylbenzothiazole;
5-{3-[4-(5-bromoindol-3-yl)-1,2,5,6-tetrahydropyrid-1-yl]propoxy}-2-methylbenzothiazole;
5-{3-[4-(4-fluoroindol-3-yl)-1,2,5,6-tetrahydropyrid-1-yl]propoxy}-2-methylbenzothiazole;
5-{4-[4-(4-fluoroindol-3-yl)-1,2,5,6-tetrahydropyrid-1-yl]butoxy}-2-methylbenzothiazole;
5-{5-[4-(4-fluoroindol-3-yl)-1,2,5,6-tetrahydropyrid-1-yl]pentoxy}-2-methylbenzothiazole;
5-{4-[4-(5-fluoroindol-3-yl)-1,2,5,6-tetrahydropyrid-1-yl]butoxy}-2-methylbenzothiazole;
5-{5-[4-(5-fluoroindol-3-yl)-1,2,5,6-tetrahydropyrid-1-yl]pentoxy}-2-methylbenzothiazole;
5-{3-[4-(7-fluoroindol-3-yl)-1,2,5,6-tetrahydropyrid-1-yl]propoxy}-2-methylbenzothiazole;
5-{4-[4-(7-fluoroindol-3-yl)-1,2,5,6-tetrahydropyrid-1-yl]butoxy}-2-methylbenzothiazole;
5-{5-[4-(7-fluoroindol-3-yl)-1,2,5,6-tetrahydropyrid-1-yl]pentoxy}-2-methylbenzothiazole;
5-{4-[4-(5-chloroindol-3-yl)-1,2,5,6-tetrahydropyrid-1-yl]butoxy}-2-methylbenzothiazole;
5-{5-[4-(5-chloroindol-3-yl)-1,2,5,6-tetrahydropyrid-1-yl]pentoxy}-2-methylbenzothiazole;
5-{3-[4-(6-chloroindol-3-yl)-1,2,5,6-tetrahydropyrid-1-yl]propoxy}-2-methylbenzothiazole;
5-{4-[4-(6-chloroindol-3-yl)1,2,5,6-tetrahydropyrid-1-yl]butoxy}-2-methylbenzothiazole;
5-{5-[4-(6-chloroindol-3-yl)-1,2,5,6-tetrahydropyrid-1-yl]pentoxy}-2-methylbenzothiazole;
5-{3-[4-(6-bromoindol-3-yl)-1,2,5,6-tetrahydropyrid-1-yl]propoxy}-2-methylbenzothiazole;
5-{4-[4-(6-bromoindol-3-yl)-1,2,5,6-tetrahydropyrid-1-yl]butoxy}-2-methylbenzothiazole;
5-{5-[4-(6-bromoindol-3-yl)-1,2,5,6-tetrahydropyrid-1-yl]pentoxy}-2-methylbenzothiazole;
5-{3-[4-(7-bromoindol-3-yl)-1,2,5,6-tetrahydropyrid-1-yl]propoxy}-2-methylbenzothiazole;
5-{4-[4-(7-bromoindol-3-yl)-1,2,5,6-tetrahydropyrid-1-yl]butoxy}-2-methylbenzothiazole;
5-{5-[4-(7-bromoindol-3-yl)-1,2,5,6-tetrahydropyrid-1-yl]pentoxy}-2-methylbenzothiazole;
5-{3-[4-(5-methoxyindol-3-yl)-1,2,5,6-tetrahydropyrid-1-yl]propoxy}-2-methylbenzothiazole;
5-{4-[4-(5-methoxyindol-3-yl)-1,2,5,6-tetrahydropyrid-1-yl]butoxy}-2-methylbenzothiazole; and
5-{5-[4-(5-methoxyindol-3-yl)-1,2,5,6-tetrahydropyrid-1-yl]pentoxy}-2-methylbenzothiazole.

4. A pharmaceutical composition comprising a compound of one of claims 1 to 3, or pharmaceutically acceptable salts and/or solvates thereof and a pharmaceutically acceptable carrier.

5. A pharmaceutical composition according to claim 4 for use in treating chronic pain, mental illnesses caused by disorders of the dopaminergic and serotonergic systems, CNS disorders, obsessive-compulsive disorders, feeding disorders, anxiety disorders, panic disorders, psychosis, acute mania, mild anxiety states, or depression.

6. A pharmaceutical composition according to claim 4 for use in treating psychosis, acute mania, mild anxiety states, or depression in combination with psychotic episodes.

7. The use of a compound of Formula I as defined below and its pharmaceutically acceptable salts for the preparation of a pharmaceutical composition for treating chronic pain, mental illnesses caused by disorders of the dopaminergic and serotonergic systems, CNS disorders, obsessive-compulsive disorders, feeding disorders, anxiety disorders, panic disorders, psychosis, acute mania, mild anxiety states, or depression: 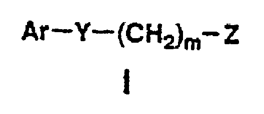 wherein
Ar is selected from 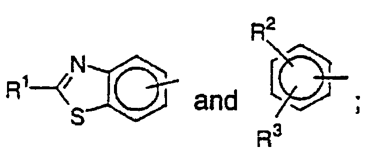
Z is II; 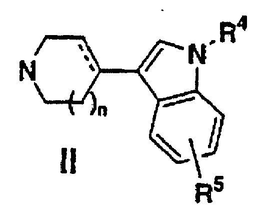
Y is sulfur or oxygen;
R¹ and R⁴ are independently selected from H and C₁₋₄ alkyl;
R² and R³ are independently selected from H, halogen, and C₁₋₄ alkoxy;
R⁵ is selected from H, halogen, C₁₋₄ alkoxy and cyano;
m is an integer from 2 to 6;
n is the integer 1; and
a dotted line represents an optional double bond.

8. The use of a compound of Formula I of claim 7 and its pharmaceutically acceptable salts for the preparation of a pharmaceutical composition for treating psychosis, acute mania, mild anxiety states, or depression in combination with psychotic episodes.

9. The use of claims 7 or 8 wherein the Formula I compound is selected from
3-[1-(3-phenoxypropyl)-4-piperidinyl]-5-cyanoindole;
3-{1-[3-(2-fluorophenoxy)propyl]-4-piperidinyl}-5-cyanoindole;
3-{1-[3-(4-fluorophenoxy)propyl]-4-piperidinyl}-5-cyanoindole;
3-{1-[3-(2-methoxyphenoxy)propyl]-4-piperidinyl}-5-cyanoindole;
3-{1-[3-(3-methoxyphenoxy)propyl]-4-piperidinyl}-5-cyanoindole;
3-{1-[3-(4-methoxyphenoxy)propyl]-4-piperidinyl}-5-cyanoindole; and
3-{1-[3-(3,4-dimethoxyphenoxy)propyl]-4-piperidinyl}-5-cyanoindole.

10. Use of a compound of one of claims 1 to 3 and its pharmaceutically acceptable salts for the preparation of a pharmaceutical composition for treating chronic pain, mental illnesses caused by disorders of the dopaminergic and serotonergic systems, CNS disorders, obsessive-compulsive disorders, feeding disorders, anxiety disorders, panic disorders, psychosis, acute mania, mild anxiety states, or depression.

11. Use of a compound of one of claims 1 to 3 and its pharmaceutically acceptable salts for the preparation of a pharmaceutical composition for treating psychosis, acute mania, mild anxiety states, or depression in combination with psychotic episodes.

12. A pharmaceutical composition comprising a compound of Formula I as defined in one of claims 7 or 9 or pharmaceutically acceptable salts and/or solvates thereof and a pharmaceutically acceptable carrier.

13. A pharmaceutical composition according to claim 12 for use in treating chronic pain, mental illnesses caused by disorders of the dopaminergic and serotonergic systems, CNS disorders, obsessive-compulsive disorders, feeding disorders, anxiety disorders, panic disorders, psychosis, acute mania, mild anxiety states, or depression.

14. A pharmaceutical composition according to claim 12 for use in treating psychosis, acute mania, mild anxiety states, or depression in combination with psychotic episodes.
